# EUROPEAN PATENT SPECIFICATION

(11) **EP 1 502 545 B1**
(45) Date of publication and mention of the grant of the patent: **13.02.2008**
(21) Application number: 04254583.0
(22) Date of filing: 30.07.2004
(51) Int. Cl.: A61B 5/06, A61B 5/07, A61B 19/00

(54) **Method and apparatus for calibrating a position sensor**
Verfahren und Vorrichtung zur Eichung eines Positionssensors
Procédé et dispositif d'étalonnage d'un capteur de position

(30) Priority: 01.08.2003 US 633299
(43) Date of publication of application: 02.02.2005
(73) Proprietor: Biosense Webster, Inc., Diamond Bar, CA 91765 (US)
(72) Inventor: Govari, Assaf, Haifa 34400 (IL)
(74) Representative: Mercer, Christopher Paul

(56) References cited:
- EP-A- 1 174 082
- WO-A-96/05768
- US-A- 5 833 608
- US-B1- 6 266 551

## Description

### FIELD OF THE INVENTION

The present invention relates generally to systems for medical diagnosis and treatment, and specifically to medical catheters whose location can be detected.

### BACKGROUND OF THE INVENTION

Various methods and devices have been described for determining the position of a probe or catheter tip inside the body using electromagnetic fields, such as in US Patent 5,391,199 to Ben-Haim, European Patent 0 776 176 to Ben-Haim et al. and corresponding US Patent Application Publication 2002/0165448 based on US Patent Application 09/273,646, US Patents 5,833,608 and 6,161,032 to Acker, and US Patents 5,558,091 and 5,752,513 to Acker et al. US Patent 5,913,820 to Bladen et al. and US Patent 5,042,486 to Pfeiler et al. also describe electromagnetic position-determination systems. Other electromagnetic tracking systems, not necessarily for medical applications, are described in US Patents 3,644,825 to Davis, Jr. et al., 3,868,565 and 4,017,858 to Kuipers, 4,054,881 to Raab, and 4,849,692 to Blood.

Because of manufacturing variations, the coils generally used in the position sensors of these position-determining systems to generate position signals may not be precisely oriented with the body of the probe. Additionally, the distance of the coils from the tip of the probe may not be precisely known, and there may be slight variations in the relative gains of the coils in response to externally-applied fields. US Patent 6,266,551 to Osadchy et al. describes methods and apparatus for pre-calibrating a probe, preferably at the time of manufacture, so as to measure and compensate for variations in the positions, orientations and gains of the coils. To calibrate the probe, a mechanical test fixture holds the probe in one or more predetermined positions and orientations, and radiators generate known, substantially uniform magnetic fields in the vicinity of the test fixture. Signals generated by the coils are analyzed and used to produce calibration data regarding the gains of the coils and deviations of the coils from orthogonality.

Various methods and devices have been described for storing, in a probe, information specific to the probe, such as calibration and identification information. These devices generally include a microchip incorporated in the probe. For example, the above-cited US Patent 6,266,551 to Osadchy et al. describes the incorporation of an electronic microcircuit in a probe, which stores information relating to calibration of the probe. Such information can include an encrypted calibration code and/or a usage code, which controls availability of the probe to a user thereof. Storage of usage codes is also described in US Patent Application Publication 2002/032380 to Acker et al., and US Patent 5,383,874 to Jackson et al. Such usage monitoring codes typically provide an indication representing the number of times the disposable device has been used or the total time the disposable device has been operatively used.

US Patent 6,370,41 to Osadchy et al. describes a catheter assembly comprising a catheter of minimal complexity and a connection cable which connects the proximal end of the catheter to a console. The catheter comprises a microcircuit which carries substantially only information specific to the catheter, such as calibration data, which is not in common with other catheters of the same model. The calibration data may be recorded in the microcircuit in the catheter in the form of lookup tables, polynomial coefficients or any other suitable form known in the art. The cable comprises an access circuit which receives the information from the catheter and passes it in a suitable form to the console.

US Patent 6,248,083 to Smith et al. describes a guide wire assembly having a measuring device mounted in the distal end portion thereof. It also has an interface cable which includes information storage, containing calibration/temperature compensation data, uniquely characteristic of the measuring device. The calibration data are used with uncompensated output from the measuring device to calculate a correct measurement value.

US Patent 5,617,857 to Chader et al. describes an imaging system which determines the location of a medical instrument. A read-only storage device is positioned on or in the medical instrument for storing initialization information characteristic of the instrument.

PCT Patent Publication WO 00/33755 to Tierney et al. describes robotic surgical tools, which include a memory. Among the memory functions is an indication of tool-specific information including measured calibration offsets, indicating misalignment of the tool drive system, tool life data, or the like.

EP-A-1174082 discloses a calibration method and associated apparatus of the type set forth in the preambles of the accompanying independent claims.

### SUMMARY OF THE INVENTION

There is provided, in accordance with an embodiment of the present invention, a method for calibrating, including:
receiving, for each of a plurality of frequencies, an indication of a characteristic value of a position sensor for placement in a patient;
measuring an actual value of the position sensor at each of the plurality of frequencies; and
determining, at each of the plurality of frequencies, calibration data indicative of a deviation of the actual value from the characteristic value, characterised in that
determining the calibration data at each of the plurality of frequencies includes calculating by subtraction a difference between the actual sensitivity and the characteristic sensitivity.

In an alternative embodiment, there is provided a method for calibrating, including:
receiving, for each of a plurality of frequencies, an indication of a characteristic value of a position sensor for placement in a patient;
measuring an actual value of the position sensor at each of the plurality of frequencies; and
determining, at each of the plurality of frequencies, calibration data indicative of a deviation of the actual value from the characteristic value, characterised in that determining, at each of the plurality of frequencies, the calibration data indicative of the deviation includes expressing the deviation as a proportion of the characteristic value.

For some applications, determining, at each of the plurality of frequencies, the calibration data indicative of the deviation includes representing the deviation in a non-linear manner with respect to the plurality of frequencies.

In an embodiment, the position sensor includes at least one coil, and determining the calibration data, at each of the plurality of frequencies, includes determining the calibration data responsive to an actual gain and a characteristic gain of the coil.

In an embodiment, the position sensor includes at least one coil, and determining the calibration data, at each of the plurality of frequencies, includes determining the calibration data responsive to at least one of: a position of the coil within the position sensor and an orientation of the coil within the position sensor, the characteristic value and the actual value at each frequency being the characteristic sensitivity and the actual sensitivity respectively.

In an embodiment, the position sensor includes a plurality of coils, and determining the calibration data, at each of the plurality of frequencies, includes determining the calibration data for each of the plurality of coils.

In an embodiment, the position sensor is incorporated in a device for placement within the patient, and determining the calibration data, at each of the plurality of frequencies, includes determining the calibration data responsive to at least one of: a position of the position sensor within the device and an orientation of the position sensor within the device the characteristic value and the actual value at each frequency being the characteristic sensitivity and the actual sensitivity respectively.

For some applications, the method includes storing the calibration data in the position sensor.

There is further provided, in accordance with an embodiment of the present invention, apparatus for calibrating a position sensor for placement in a patient, the apparatus including:
a test fixture, adapted to hold the position sensor in a known position and orientation;
a plurality of radiator coils, adapted to generate fields at a plurality of frequencies; and
a computer, adapted to:
   receive, for each of the plurality of frequencies, an indication of a characteristic value of the position sensor,
   measure an actual value of the position sensor, responsive to the fields generated at each of the plurality of frequencies, and
   determine, at each of the plurality of frequencies, calibration data indicative of a deviation of the actual value from the characteristic value,
characterised in that the computer is adapted to determine the calibration data, at each of the plurality of frequencies, by calculating subtraction a difference between the actual value and the characteristic value.

There is yet further provided, in accordance with an alternative embodiment of the present invention, apparatus for calibrating a position sensor for placement in a patient, the apparatus including:
a test fixture, adapted to hold the position sensor in a known position and orientation;
a plurality of radiator coils, adapted to generate fields at a plurality of frequencies; and
a computer, adapted to:
   receive, for each of the plurality of frequencies, an indication of a characteristic value of the position sensor,
   measure an actual value of the position sensor, responsive to the fields generated at each of the plurality of frequencies, and
   determine, at each of the plurality of frequencies, calibration data indicative of a deviation of the actual value from the characteristic value,
characterised in that the computer is adapted to determine, at each of the plurality of frequencies, the calibration data indicative of the deviation by expressing the deviation as a proportion of the characteristic value.

### BRIEF DESCRIPTION OF THE DRAWINGS

The present invention will be more fully understood from the following detailed description of embodiments thereof, taken together with the drawings in which:
Fig. 1 is a simplified pictorial illustration of a system including a catheter, in accordance with an embodiment of the present invention;
Fig. 2 is a schematic, pictorial illustration of the distal end of the catheter of Fig. 1. in accordance with an embodiment of the present invention;
Fig. 3 is a detailed schematic view of a connector at the proximal end of the catheter, in accordance with an embodiment of the present invention;
Fig. 4 is a graph of sensitivity vs. frequency for a coil in the catheter of Fig. 1, in accordance with an embodiment of the present invention;
Fig. 5 is a flow chart that schematically illustrates a method for manipulating and storing calibration data of a coil of the catheter of Fig. 1, in accordance with an embodiment of the present invention; and
Fig. 6 is a schematic, pictorial illustration of an implantable or insertable encapsulated transponder 600, in accordance with an embodiment of the present invention.

### DETAILED DESCRIPTION OF EMBODIMENTS

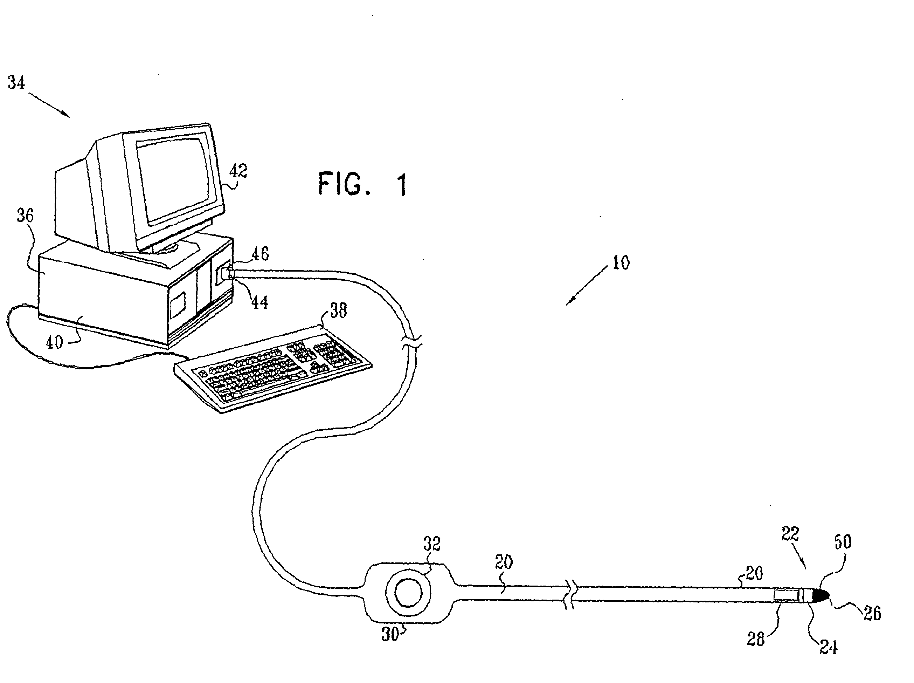

Fig. 1 is a simplified pictorial illustration of a catheter system 10 comprising an elongate probe, such as a catheter 20, for insertion into the human body, in accordance with an embodiment of the present invention. It is to be understood that although the following embodiments are described with reference to a catheter, embodiments of the present invention are equally applicable to use with other types of probes.

Catheter 20 typically includes a handle 30 for operation of the catheter by a user. Controls 32 on handle 30 enable the user to steer a distal end 22 of the catheter in a desired direction, or to position and/or orient distal end 22 as desired.

System 10 further comprises a console 34, which enables the user to observe and regulate the functions of catheter 20. Console 34 typically includes a computer 36, a keyboard 38, signal processing circuits 40, which are typically inside the computer, and a display 42. Signal processing circuits 40 typically receive, amplify, filter, and digitize signals from catheter 20, whereupon these digitized signals are received and used by computer 36 to compute the position and orientation of the catheter. Catheter 20 is typically coupled at its proximal end by a connector 44 to a mating receptacle 46 on console 34.

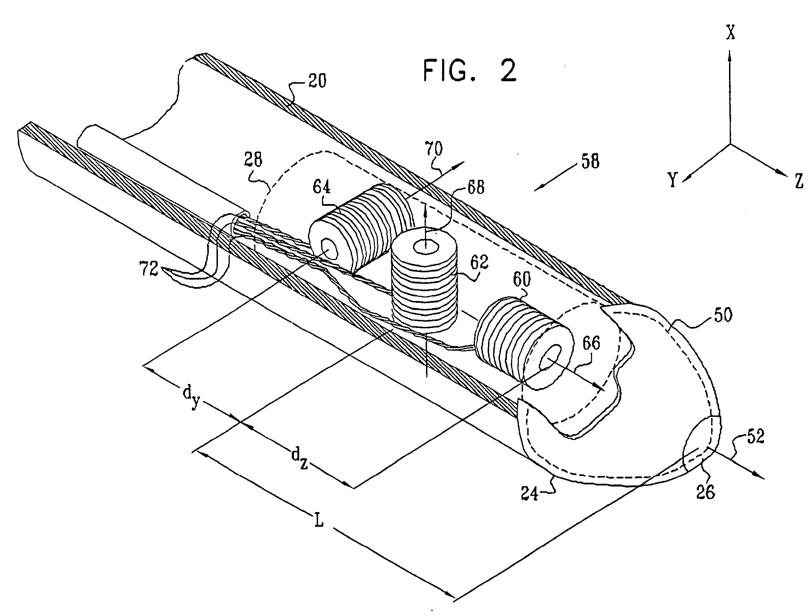

Fig. 2 is a schematic, pictorial illustration of distal end 22 of catheter 20, in accordance with an embodiment of the present invention. Distal end 22 comprises a functional portion 24 for performing diagnostic and/or therapeutic functions, adjacent to a distal tip 26 of the catheter. Functional portion 24 typically comprises a tool 50, such as an ultrasound transducer or ablation electrode. Distal end 22 of catheter 20 further comprises a position sensing device 28 that generates signals used to determine the position and orientation of the catheter within the body. Position sensing device 28 is typically adjacent to functional portion 24. There is typically a fixed positional and orientational relationship between position sensing device 28 and portion 24.

Position sensing device 28 typically comprises one or more coils 58, e.g., three non-concentric coils 60, 62 and 64, such as described in the above-cited European Patent 0 776 176 to Ben-Haim et al. This device enables continuous generation of six dimensions of position and orientation information. Coils 60, 62 and 64 have respective axes 66, 68 and 70 which typically, but not necessarily, define orthogonal Cartesian axes Z, X and Y, respectively, as shown in Fig. 2, wherein the Z-axis is parallel to the long axis of catheter 20 and the X- and Y-axes define a plane perpendicular thereto. The coils each have a fixed position and orientation with respect to each other.

Although embodiments of the present invention are described herein with reference to the position signal generating device shown in Fig. 2 and described above, it is to be understood that the inventive concepts of the present invention are similarly applicable to probes including other position sensing devices. For example, embodiments of the present invention may comprise a single coil for generating position signals, or two or more such coils, which may be concentric or non-concentric. Other embodiments of the present invention may comprise other types of position sensing devices, such as Hall effect devices.

As shown in Fig. 2, position sensing device 28 is located in catheter 20 at a distance L from distal tip 26, where L is defined herein for convenience as the distance along the Z-axis from central axis 68 of coil 62 to tip 26. Respective axes 66 and 70 of coils 60 and 64 are displaced from axis 68 by respective distances dy and dz.

Signal processing circuits 40 in console 34 receive signals carried by coil wires 72 from coils 60, 62, and 64, and convey them to computer 36, which computes the three-dimensional translational position of position sensing device 28 and the rotational orientation of axes 66, 68 and 70, relative to a fixed, external coordinate frame. The actual position and orientation of distal tip 26 are then computed by taking into account the distance L of tip 26 from the center of position sensing device 28, as defined by axis 68, and the orientation of axes 66, 68 and 70.

It has been found empirically that because of deviations in the process of manufacturing catheter 20 and coils 58, variations in the respective gains of the coils may cause errors in determination of position and orientation of the catheter. In addition, the distance L typically varies from one catheter to another, leading to errors in calculating the position of tip 26. Furthermore, axis 66 of coil 60 typically deviates from absolute alignment with the long axis of catheter 20, which passes through tip 26. Moreover, axes 68 and 70 of coils 62 and 64 respectively are typically not precisely orthogonal to axis 66 or to each other, thereby inducing additional errors in determination of position and orientation of the catheter. Still furthermore, there are typically variations in the distances dy and dz.

Therefore, in an embodiment of the present invention, position sensing device 28 is calibrated, taking into account variations in the respective gains of the coils, and, optionally, one or more of the other variations described above, before the catheter is inserted into a patient's body. In an embodiment, this calibration is performed using one or more test fixtures (not shown) and methods of calibration described in the above-mentioned US Patent 6,266,551 to Osadchy et al. For example, the '551 patent describes a jig comprising three mutually orthogonal pairs of parallel radiator coils mounted on a base. Each radiator coil pair generates a predetermined, substantially uniform magnetic field that is substantially normal to the planes defined by the pair of coils, and is thus substantially orthogonal to fields generated by the other two radiator coil pairs. In order to calibrate the gains of the coils, total amplitudes of the respective catheter coil signals are derived by summing the squares of the amplitudes of the signals generated by each of the catheter coils in response to each of the radiator coil pairs in turn. Since the magnetic fields in the vicinity of coils 60, 62 and 64 have equal and substantially uniform components along each of the coil axes 66, 68 and 70, the total signal amplitudes will be independent of the respective orientations and positions of coils 60, 62 and 64, and will depend only on the respective coil gains.

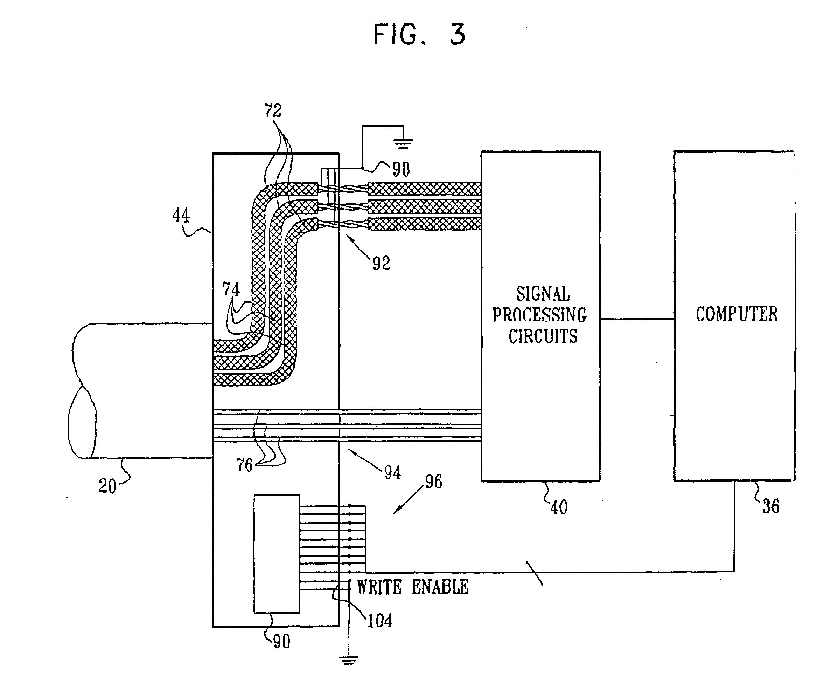

Reference is now made to Fig. 3, which is a detailed schematic view of connector 44 at the proximal end of catheter 20, in accordance with an embodiment of the present invention. For some applications, connector 44 comprises a digital microcircuit 90, comprising a memory 91 in which calibration data for catheter 20 are electronically stored. Memory 91 comprises an EEPROM, an EPROM, a PROM, a Flash ROM, non-volatile RAM, or other type of programmable memory known in the art. Alternatively, microcircuit 90 is located elsewhere in catheter 20, rather than in connector 44. For example, the microcircuit may be located in a vicinity of handle 30.

In the embodiment shown in Fig. 3, connector 44 comprises pins 92, 94, 96, and 98, which mate with corresponding sockets in receptacle 46 on console 34 (Fig. 1). Typically, functional pins 94 carry analog electrophysiological signals conveyed over functional wires 76 to signal processing circuits 40. Coil pins 92 typically carry position and orientation signals conveyed by coil wires 72 from coils 58 to signal processing circuits 40 and computer 36, which computes the position and orientation of catheter 20. The computer further reads digital calibration correction function data stored in microcircuit 90 via memory pins 96, and uses these data in computing the correct catheter position and orientation. In embodiments in which microcircuit 90 is located elsewhere in catheter 20, the microcircuit is typically coupled to memory pins 96 over a set of wires (not shown).

For some applications, one or more write-enable pins 104 are likewise coupled to microcircuit 90. These pins are used to enable programming of the microcircuit with the desired calibration data. At the time of calibration, the write-enable input is enabled, and calibration data are recorded in the microcircuit. Thereafter the write-enable input is disabled, for example by removing the write-enable pin or by connecting it to electrical ground, as shown in Fig. 3, so that further calibration data may not be recorded in the microcircuit, and the microcircuit functions in a read-only mode.

Alternatively, in embodiments of the present invention wherein microcircuit 90 comprises an EEPROM device, the write-enable input may be disabled by sending a write-protect command to the device. This command may be reversible or irreversible.

In an embodiment of the present invention, microcircuit 90 comprises a device incorporating password-secured access control, and write-access to the microcircuit requires that an appropriate password first be entered. The microcircuit is programmed with calibration data at the time of manufacture, and thereafter operates in a "read access only" mode, with all write operations locked out, or in a "read access and program only" mode, in which certain data, but not calibration data, may be written to the device. Changing the mode of operation of the microcircuit requires that an appropriate password be entered, which password is generally unavailable to users of the system.

In an embodiment of the present invention, microcircuit 90 comprises an EPROM or PROM device, which is contained in the catheter connector, and the input and output connections of the EPROM or PROM are coupled to pins of the connector. Calibration data are recorded in the EPROM or PROM at the time of manufacture using a suitable programming device, not shown in the figures, which receives data from the computer used in calibration. The programming device is connected to catheter connector 44 and programs the EPROM or PROM by inputting digital signals thereto through the connector. Thereafter, the EPROM or PROM may not be re-programmed.

In some embodiments of the present invention, data recorded in microcircuit 90 include a calibration code, which is encrypted in accordance with methods known in the art, so as to ensure that the calibration data have not been altered or corrupted. The calibration code typically includes a checksum. When the user connects catheter 20 to console 34, computer 36 reads the calibration code and compares the code with pre-programmed values. If the code does not match the desired pre-programmed value, the computer causes a message to be displayed by display 42 indicating that the catheter may not be appropriately calibrated. The computer may further cause the system to cease operation until a catheter having a code matching the desired pre-programmed value is connected thereto.

Typically, the calibration code is encrypted using a method that prevents decryption by unauthorized parties, for example the RSA encryption scheme, using a public key and a private key, or other methods known in the art. When a method such as RSA encryption is used, the private key is known only to authorized manufacturers of the catheter, so as to prevent the possible use of unauthorized substitutes of possibly inferior quality.

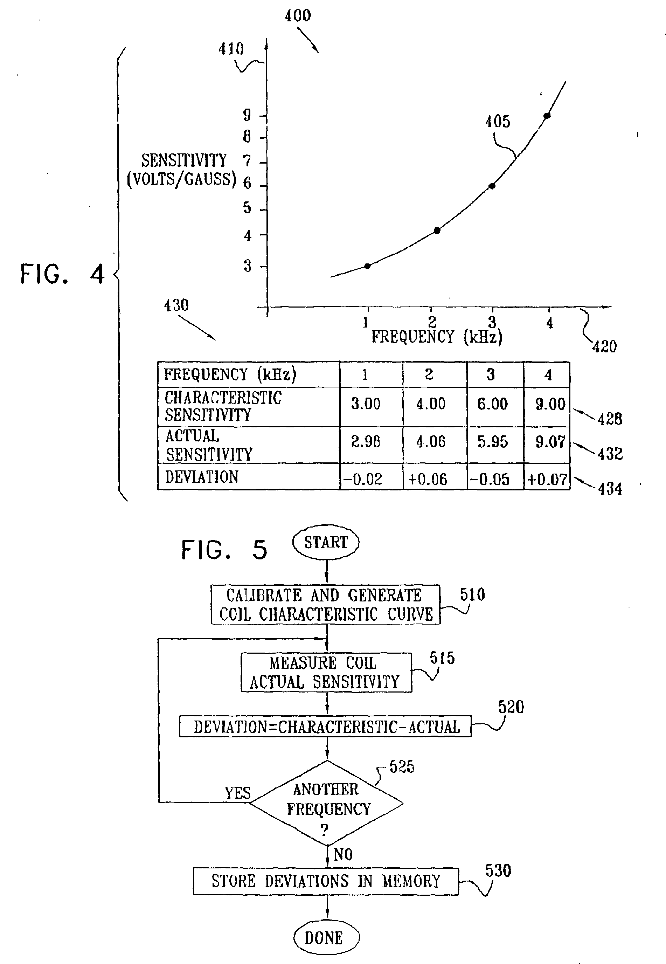

Reference is now made to Fig. 4, which is a graph 400 of sensitivity vs. frequency for one of coils 58, in accordance with an embodiment of the present invention. Each type of coil has a characteristic curve 405, which represents the sensitivity of the coil at different field frequencies. A horizontal axis 420 of graph 400 indicates frequency (typically expressed in kHz), while a vertical axis 410 of the graph represents the sensitivity of the coil (typically expressed in volts/gauss) at each frequency. The sensitivity values are typically calculated based on the design of the coil, or empirically determined for a large number of frequencies. For example, in the characteristic curve shown in Fig. 4, the coil exhibits a calculated sensitivity of 3.00 volts/gauss at a frequency of 1 kHz, and of 6.00 volts/gauss at 3 kHz. Depending on the type of coil, the characteristic curve may be non-linear, as shown in Fig. 4, or linear.

In practice, because of manufacturing variances, as described hereinabove, a specific coil 58 often has sensitivities at each frequency that differ from the values of characteristic curve 405. A row 428 of a table 430 shows exemplary characteristic sensitivity values, i.e., expected values, at several frequencies (indicated immediately above the table on axis 420), while a second row 432 of the table shows exemplary actual measured sensitivity values for the coil. For example, at 1 kHz, the actual sensitivity, as determined during a calibration procedure, is 2.98 volts/gauss, which is 0.02 volts/gauss less than the calculated sensitivity of 3.00 volts/gauss. For each frequency represented in table 430, the deviation between characteristic sensitivity and actual sensitivity is indicated in a row 434. The coil is calibrated for each frequency for which it is desirable to store calibration data. Optionally, such calibration is performed using procedures described in the above-mentioned US Patent 6,266,551 to Osadchy et al.

Fig. 5 is a flow chart that schematically illustrates a method for manipulating and storing calibration data of one of coils 58 in memory 91, in accordance with an embodiment of the present invention. A characteristic curve, representing theoretical sensitivities of ideal coils of the same type as coil 58, is generated, as described hereinabove, at a characteristic curve generation step 510. As part of a typical manufacturing process, one or more coils 58 are then installed in position sensing device 28 and the actual sensitivity of each of the coils is measured at a chosen frequency, at an actual sensitivity measurement step 515, as described hereinabove. Alternatively, the actual sensitivity of each coil is measured prior to installation of the coil in position sensing device 28. A deviation is calculated for each coil, typically by subtracting the actual sensitivity from the characteristic sensitivity, at a deviation calculation step 520. If there are additional frequencies at which it is desired to calibrate coils 58, as determined at an additional frequency check step 525, the method returns to step 515.

On the other hand, if the coils have been calibrated at all desired frequencies, respective deviations are stored in memory 91, as described hereinbelow, at a store deviation step 530. For some applications, additional calibration data are stored at step 530, e.g., data relating to the position and/or orientation of one or more coils in the catheter, and/or data relating to the position and/or orientation of tool 50.

At step 530, the calculated deviations, rather than the actual sensitivities, are stored in memory 91, thereby reducing the memory consumption. Based on the range of acceptable manufacturing tolerances, a predetermined number of bits of memory is typically allocated in memory 91 for each frequency. For example, if the deviation values have a range of +0.08 to -0.07 volts/gauss, sixteen possible deviation values (including 0.00, representing no deviation) can be stored in four bits (a nibble) of data, for a single coil at a single frequency. Alternatively, a non-linear representation of the deviations is used, in which, for example, values closer to 0.00 are allotted more bits, thereby providing greater precision in the representation of these values. Further alternatively, each deviation is expressed as a percentage value of the characteristic sensitivity or other proportion of the characteristic sensitivity, and an appropriate number of bits is selected to represent the deviation. Such a percentage calculation may be appropriate, for example, in applications in which absolute deviation values are generally greater at higher frequencies, but the deviations, when expressed as a percentage of the characteristic sensitivity, are generally constant across the frequencies. Other methods of calculating the deviation will be readily apparent to those skilled in the art, having read the present patent application, and are considered within the scope of the present invention.

During use of the probe during a procedure, the amplitudes of signals received from these coils are adjusted using the calibration data stored in memory 91.

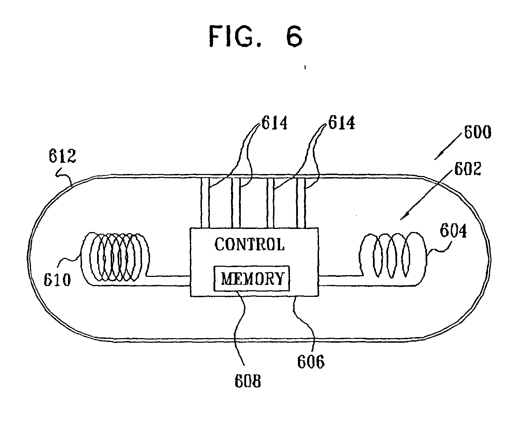

Reference is now made to Fig. 6, which is a schematic, pictorial illustration of an implantable or insertable encapsulated transponder 600, in accordance with an embodiment of the present invention. Transponder 600 comprises a position sensing device 602 that generates signals used to determine the position and orientation of the transponder within the body. Position sensing device 602 is typically similar to position sensing device 28, and typically comprises one or more sensing coils 604 similar to coils 58. Transponder 600 further comprises a control chip 606, which is typically similar to control chip 44. Control chip 606 comprises a memory 608, generally similar to memory 91. Transponder 600 typically additionally comprises a coil 610, for receiving externally-generated power and transmitting position information, using analog or digital signals. Transponder 600 may utilize techniques described in EP 1321097 to Govari. (Position sensing device 28, coils 58, control chip 44, and memory 91 are described hereinabove with reference to Fig. 2.)

Transponder 600 is typically encapsulated in sealed packaging 612, such as ceramic packaging. For some applications, control chip 606 comprises electronic contact pins 614, which enable the control chip to be programmed from an external device. Typically, sealed packaging 612 covers the pins, which are only used for initialization of transponder 600 during manufacture and calibration prior to sealing.

In an embodiment of the present invention, position sensing device 602 is calibrated before transponder 600 is inserted into a patient's body, using the calibration and data storage techniques described hereinabove, *mutatis mutandis.* For some applications, the position sensing device is calibrated prior to application of sealed packaging 612, in which case pins 614 are typically used to transmit the calibration data for storage in memory 608. Alternatively, the calibration is performed after transponder 600 has been sealed, and the calibration data are transmitted wirelessly to coil 610 or coil 604 for storage in memory 608.

It will thus be appreciated that the embodiments described above are cited by way of example, and that the present invention is limited to the appended claims.

## Claims

1. A method for calibrating, comprising:
receiving (510), for each of a plurality of frequencies, an indication of a characteristic value (428) of a position sensor (28) for placement in a patient;
measuring (515) an actual value (432) of the position sensor at each of the plurality of frequencies; and
determining (520), at each of the plurality of frequencies, calibration data (434) indicative of a deviation of the actual value from the characteristic value,
**characterised in that** determining the calibration data (434) at each of the plurality of frequencies comprises calculating by subtraction (520) a difference between the actual value (432) and the characteristic value (428).

2. A method for calibrating, comprising:
receiving (510), for each of a plurality of frequencies,an indication of a characteristic value (428) of a position sensor (28) for placement in a patient;
measuring (515) an actual value (432) of the position sensor at each of the plurality of frequencies; and
determining (520), at each of the plurality of frequencies, calibration data (434) indicative of a deviation of the actual value from the characteristic value,
**characterised in that** determining (520), at each of the plurality of frequencies, the calibration data indicative of the deviation comprises expressing the deviation as a proportion of the characteristic value (428).

3. The method according to claim 1 or claim 2, wherein determining (520), at each of the plurality of frequencies, the calibration data (434) indicative of the deviation comprises representing the deviation in a non-linear manner with respect to the plurality of frequencies.

4. The method according to claim 12 or 3, wherein the position sensor (28) includes at least one coil (60; 62; 64), and wherein determining (520) the calibration data (434), at each of the plurality of frequencies, comprises determining the calibration data responsive to an actual gain and a characteristic gain of the coil.

5. The method according to claim 1, 2 or 3, wherein the position sensor (28) includes at least one coil (60; 62; 64), and wherein determining (520) the calibration data (434), at each of the plurality of frequencies, comprises determining the calibration data responsive to at least one of: a position of the coil within the position sensor and an orientation of the coil within the position sensor, the characteristic value and the actual value at each frequency being the characteristic sensitivity (428) and the actual sensitivity (432) respectively.

6. The method according to any preceding claim, wherein the position sensor (28) includes a plurality of coils (60; 62; 64), and wherein determining (520) the calibration data (434), at each of the plurality of frequencies, comprises determining the calibration data for each of the plurality of coils.

7. The method according to any preceding claim, wherein the position sensor (28) is incorporated in a device for placement within the patient, and wherein determining (520) the calibration data (434), at each of the plurality of frequencies, comprises determining the calibration data responsive to at least one of: a position of the position sensor (28) within the device and an orientation of the position sensor within the device, the characteristic value and the actual value at each frequency being the characteristic sensitivity (428) and the actual sensitivity (432) respectively.

8. The method according to any preceding claim, further comprising storing (530) the calibration data (434) in the position sensor (28).

9. Apparatus for calibrating a position sensor (28) for placement in a patient, the apparatus comprising:
a test fixture, adapted to hold the position sensor in a known position and orientation;
a plurality of radiator coils, adapted to generate fields at a plurality of frequencies; and
a computer (36), adapted to:
receive, for each of the plurality of frequencies, an indication of a characteristic value (428) of the position sensor,
measure an actual value (432) of the position sensor, responsive to the fields generated at each of the plurality of frequencies, and
determine (520), at each of the plurality of frequencies, calibration data (434) indicative of a deviation of the actual value from the characteristic value,
**characterised in that** the computer (36) is adapted to determine the calibration data (434), at each of the plurality of frequencies, by calculating by subtraction (520) a difference between the actual value (432) and the characteristic value (428).

10. Apparatus for calibrating a position sensor (28) for placement in a patient, the apparatus comprising:
a test fixture, adapted to hold the position sensor in a known position and orientation;
a plurality of radiator coils, adapted to generate fields at a plurality of frequencies; and
a computer (36), adapted to:
receive, for each of the plurality of frequencies an indication of a characteristic value (428) of the position sensor,
measure an actual value (432) of the position sensor, responsive to the fields generated at each of the plurality of frequencies, and
determine (520), at each of the plurality of frequencies, calibration data (434) indicative of a deviation of the actual value from the characteristic value,
**characterised in that** the computer (36) is adapted to determine (520), at each of the plurality of frequencies, the calibration data (434) indicative of the deviation by expressing the deviation as a proportion of the characteristic value (428).

11. The apparatus according to claim 9 or claim 10. wherein the computer (36) is adapted to determine (520), at each of the plurality of frequencies, the calibration data (434) indicative of the deviation by representing the deviation in a non-linear manner with respect to the plurality of frequencies.

12. The apparatus according to claim 9, 10or 11, wherein the position sensor (28) includes at least one coil (60; 62; 64), and wherein the computer is adapted to determine (520) the calibration data (434), at each of the plurality of frequencies, responsive to an actual gain and a characteristic gain of the coil.

13. The apparatus according to claim 9. 10 or 11, wherein the position sensor (28) includes at least one coil (60; 62; 64), and wherein the computer is adapted to determine (520) the calibration data at each of the plurality of frequencies, responsive to at least one of: a position and of the coil within the position sensor an orientation of the coil within the position sensor, the characteristic value and the actual value at each frequency being the characteristic sensitivity (428) and the actual sensitivity (432) respectively.

14. The apparatus according to any of claims 9 to 13, wherein the position sensor (28) includes a plurality of coils (60; 62; 64), and wherein the computer (36) is adapted to determine, at each of the plurality of frequencies, the calibration data (434) for each of the plurality of coils.

15. The apparatus according to any of claims 9 to 14, wherein the position sensor (28) is incorporated in a device for placement in the patient, and wherein the computer is adapted to determine (520) the calibration data (434), at each of the plurality of frequencies, responsive to at least one of: a position of the position sensor within the device and an orientation of the position sensor within the device, the characteristic value and the actual value at each frequency being the characteristic sensitivity (428) and the actual sensitivity (432) respectively.

16. The apparatus according to any of claims 9 to 15, wherein the computer (36) is adapted to store (530) the calibration data in the position sensor (28).

17. The apparatus according to claim 16, further comprising a device adapted to be placed into a patient, the device comprising:
the position sensor (28) ; and
a memory (91) adapted to store calibration data (434).

18. The apparatus according to claim 17, wherein the device is adapted to be incorporated in an elongate probe (20).

19. The apparatus according to claim 17, wherein the device is adapted to be incorporated in a capsule (600), adapted to be placed in the patient.

## Patentansprüche

1. Kalibrierungsverfahren, das folgendes umfaßt:
Empfangen (510) einer Angabe eines charakteristischen Werts (428) eines Positionssensors (28) zum Plazieren in einem Patienten für jede von mehreren Frequenzen;
Messen (515) des tatsächlichen Wertes (432) des Positionssensors bei jeder der mehreren Frequenzen; und
Bestimmen (520) von Kalibrierungsdaten (434), welche die Abweichung des tatsächlichen Wertes von dem charakteristischen Wert angeben, bei jeder der mehreren Frequenzen,
**dadurch gekennzeichnet, daß** das Bestimmen der Kalibrierungsdaten (434) bei jeder der mehreren Frequenzen ein Berechnen einer Differenz zwischen dem tatsächlichen Wert (432) und dem charakteristischen Wert (428) durch Subtraktion (520) umfaßt.

2. Kalibrierungsverfahren, das folgendes umfaßt:
Empfangen (510) einer Angabe eines charakteristischen Werts (428) eines Positionssensors (28) zum Plazieren in einem Patienten für jede von mehreren Frequenzen;
Messen (515) des tatsächlichen Wertes (432) des Positionssensors an jeder der mehreren Frequenzen; und
Bestimmen (520) von Kalibrierungsdaten (434), welche die Abweichung des tatsächlichen Wertes von dem charakteristischen Wert angeben, bei jeder der mehreren Frequenzen,
**dadurch gekennzeichnet, daß** das Bestimmen (520) der Kalibrierungsdaten, welche die Abweichung angeben, bei jeder der mehreren Frequenzen ein Ausdrücken der Abweichung als ein Anteil des charakteristischen Werts (428) umfaßt.

3. Verfahren nach Anspruch 1 oder 2, wobei das Bestimmen (520) der Kalibrierungsdaten (434), welche die Abweichungen angeben, bei jeder der mehreren Frequenzen ein Darstellen der Abweichung in einer nicht linearen Weise in bezug auf die mehreren Frequenzen umfaßt.

4. Verfahren nach Anspruch 1, 2 oder 3, wobei der Positionssensor (28) wenigstens eine Spule (60; 62; 64) umfaßt, und wobei das Bestimmen (520) der Kalibrierungsdaten (434) bei jeder der mehreren Frequenzen ein Bestimmen der Kalibrierungsdaten umfaßt, welche auf eine tatsächliche Verstärkung und eine charakteristische Verstärkung der Spule reagieren.

5. Verfahren nach Anspruch 1, 2 oder 3, wobei der Positionssensor (28) wenigstens eine Spule (60; 62; 64) umfaßt, und wobei das Bestimmen (520) der Kalibrierungsdaten (434) bei jeder der mehreren Frequenzen das Bestimmen der Kalibrierungsdaten umfaßt, die auf wenigstens eines von folgendem reagieren: eine Position der Spule in dem Positionssensor und eine Orientierung der Spule in dem Positionssensor, wobei der charakteristische Wert und der tatsächliche Wert bei jeder Frequenz die charakteristische Empfindlichkeit (428) bzw. die tatsächliche Empfindlichkeit (432) sind.

6. Verfahren nach einem der vorhergehenden Ansprüche, wobei der Positionssensor (28) mehrere Spulen (60; 62; 64) umfaßt, und wobei das Bestimmen (520) der Kalibrierungsdaten (434) bei jeder der mehreren Frequenzen ein Bestimmen der Kalibrierungsdaten für jede der mehreren Spulen umfaßt.

7. Verfahren nach einem der vorhergehenden Ansprüche, wobei der Positionssensor (28) in einer Vorrichtung zum Plazieren in einem Patienten eingebaut ist, und wobei das Bestimmen (520) der Kalibrierungsdaten (434) bei jeder der mehreren Frequenzen das Bestimmen der Kalibrierungsdaten umfaßt, die auf wenigstens eines von folgendem reagieren: eine Position des Positionssensors (28) in der Vorrichtung und eine Orientierung des Positionssensors in der Vorrichtung, wobei der charakteristische Wert und der tatsächliche Wert bei jeder Frequenz die charakteristische Empfindlichkeit (428) bzw. die tatsächliche Empfindlichkeit (432) ist.

8. Verfahren nach einem der vorhergehenden Ansprüche, das weiterhin ein Speichern (530) der Kalibrierungsdaten (434) in dem Positionssensor (28) umfaßt.

9. Gerät zum Kalibrieren eines Positionssensors (28) zur Plazierung in einem Patienten, wobei das Gerät folgendes aufweist:
eine Testaufnahme, die dafür eingerichtet ist, den Positionssensor in einer bekannten Position und Orientierung zu halten;
mehrere Radiatorspuren, die dafür eingerichtet sind, Felder bei mehreren Frequenzen zu erzeugen; und
einen Computer (36), der zu folgendem eingerichtet ist:
Empfangen für jede der mehreren Frequenzen einer Angabe eines charakteristischen Wertes (428) des Positionssensors,
Messen eines tatsächlichen Wertes (432) des Positionssensors, der auf die Felder reagiert, die bei jeder der mehreren Frequenzen erzeugt werden, und
Bestimmen (520) von Kalibrierungsdaten (434), welche die Abweichung des tatsächlichen Werts von dem charakteristischen Wert angeben, bei jeder der mehreren Frequenzen,
**dadurch gekennzeichnet, daß** der Computer (36) dafür eingerichtet ist, die Kalibrierungsdaten (434) bei jeder der mehreren Frequenzen durch Berechnung zu bestimmen, indem eine Differenz zwischen dem tatsächlichen Wert (432) und dem charakteristischen Wert (428) subtrahiert wird (520).

10. Gerät zum Kalibrieren eines Positionssensors (28) zur Plazierung in einem Patienten, wobei das Gerät folgendes aufweist:
eine Testaufnahme, die dafür eingerichtet ist, den Positionssensor in einer bekannten Position und Orientierung zu halten;
mehrere Radiatorspuren, die dafür eingerichtet sind, Felder bei mehreren Frequenzen zu erzeugen; und
einen Computer (36), der zu folgendem eingerichtet ist:
Empfangen für jede der mehreren Frequenzen einer Angabe eines charakteristischen Wertes (428) des Positionssensors,
Messen eines tatsächlichen Wertes (432) des Positionssensors, der auf die Felder reagiert, die bei jeder der mehreren Frequenzen erzeugt werden, und
Bestimmen (520) von Kalibrierungsdaten (434), welche die Abweichung des tatsächlichen Werts von dem charakteristischen Wert angeben, bei jeder der mehreren Frequenzen,
**dadurch gekennzeichnet, daß** der Computer (36) dafür eingerichtet ist, bei jeder der Frequenzen die Kalibrierungsdaten (434), welche die Abweichung angeben, durch Ausdrücken der Abweichung als ein Anteil des charakteristischen Wertes (428) zu bestimmen (520).

11. Gerät nach Anspruch 9 oder 10, wobei der Computer (36) dafür eingerichtet ist, bei jeder der mehreren Frequenzen die Kalibrierungsdaten (434), welche die Abweichung angeben, durch Darstellen der Abweichung in einer nicht linearen Weise in bezug auf die mehreren Frequenzen zu bestimmen (520).

12. Gerät nach Anspruch 9, 10 oder 11, wobei der Positionssensor (28) wenigstens eine Spule (60; 62; 64) umfaßt, und wobei der Computer dafür eingerichtet ist, die Kalibrierungsdaten (434) zu bestimmen (520), welche auf eine tatsächliche Verstärkung bei jeder der mehreren Frequenzen und eine charakteristische Verstärkung der Spule reagieren.

13. Gerät nach Anspruch 9, 10 oder 11, wobei der Positionssensor (28) wenigstens eine Spule (60; 62; 64) umfaßt, und wobei der Computer dafür eingerichtet ist, die Kalibrierungsdaten bei jeder der mehreren Frequenzen zu bestimmen (520), welche auf wenigstens eines von folgendem reagieren: eine Position der Spule in dem Positionssensor und eine Orientierung der Spule in dem Positionssensor, wobei der charakteristische Wert und der tatsächliche Wert bei jeder Frequenz, die charakteristische Empfindlichkeit (428) bzw. die tatsächliche Empfindlichkeit (432) sind.

14. Gerät nach einem der Ansprüche 9 bis 13, wobei der Positionssensor (28) mehrere Spulen (60; 62; 64) aufweist, und wobei der Computer (36) dafür eingerichtet ist, bei jeder der mehreren Frequenzen die Kalibrierungsdaten (434) für jede der mehreren Spulen zu bestimmen.

15. Gerät nach einem der Ansprüche 9 bis 14, wobei der Positionssensor (28) in einer Vorrichtung zum Plazieren in einem Patienten eingebaut ist, und wobei der Computer dafür eingerichtet ist, die Kalibrierungsdaten (434) bei jeder der mehreren Frequenzen zu bestimmen (520), welche auf wenigstens eines von folgendem reagieren: eine Position des Positionssensors in der Vorrichtung und eine Orientierung des Positionssensors in der Vorrichtung, wobei der charakteristische Wert und der tatsächliche Wert bei jeder Frequenz die charakteristische Empfindlichkeit (428) bzw. die tatsächliche Empfindlichkeit (432) sind.

16. Gerät nach einem der Ansprüche 9 bis 15, wobei der Computer (36) dafür eingerichtet ist, die Kalibrierungsdaten in den Positionssensor (28) zu speichern (530).

17. Gerät nach Anspruch 16, das weiterhin eine Vorrichtung umfaßt, die dafür eingerichtet ist, in einem Patienten plaziert zu werden, wobei die Vorrichtung folgendes aufweist:
einen Positionssensor (28); und
einen Speicher (91), der dafür eingerichtet ist, Kalibrierungsdaten (434) zu speichern.

18. Gerät nach Anspruch 17, wobei die Vorrichtung dafür eingerichtet ist, in einer länglichen Sonde (20) eingebaut zu werden.

19. Gerät nach Anspruch 17, wobei die Vorrichtung dafür eingerichtet ist, in einer Kapsel (600) eingebaut zu werden, die dafür eingerichtet ist, in einem Patienten plaziert zu werden.

## Revendications

1. Procédé d'étalonnage, comprenant les étapes consistant à :
recevoir (510), pour chacune d'une pluralité de fréquences, une indication d'une valeur caractéristique (428) d'un capteur de position (28) destiné à être placé chez un patient ;
mesurer (515) une valeur réelle (432) du capteur de position au niveau de chacune de la pluralité de fréquences ; et
déterminer (520), au niveau de chacune de la pluralité de fréquences, des données d'étalonnage (434) indicatrices d'un écart de la valeur réelle par rapport à la valeur caractéristique,
**caractérisé en ce que** la détermination des données d'étalonnage (434) au niveau de chacune de la pluralité de fréquences comprend l'étape consistant à calculer par soustraction (520) une différence entre la valeur réelle (432) et la valeur caractéristique (428).

2. Procédé d'étalonnage comprenant les étapes consistant à :
recevoir (510), pour chacune d' une pluralité de fréquences, une indication d'une valeur caractéristique (428) d'un capteur de position (28) destiné à être placé chez un patient ;
mesurer (515) une valeur réelle (432) du capteur de position au niveau de chacune de la pluralité de fréquences ; et
déterminer (520), au niveau de chacune de la pluralité de fréquences, des données d'étalonnage (434) indicatrices d'un écart de la valeur réelle par rapport à la valeur caractéristique,
**caractérisé en ce que** la détermination (520), au niveau de chacune de la pluralité de fréquences, des données d'étalonnage indicatrices de l'écart comprend l'étape consistant à exprimer l'écart en tant qu'une proportion de la valeur caractéristique (428).

3. Procédé selon la revendication 1, ou 2, dans lequel la détermination (520), au niveau de chacune de la pluralité de fréquences, des données d'étalonnage (434) indicatrices de l'écart comprend l'étape consistant à représenter l'écart de manière non linéaire en rapport avec la pluralité de fréquences.

4. Procédé selon la revendication 1, 2 ou 3, dans lequel le capteur de position (28) comporte au moins une bobine (60 ; 62 ; 64), et dans lequel la détermination (520) des données d'étalonnage (434), au niveau de chacune de la pluralité de fréquences, comprend l'étape consistant à déterminer les données d'étalonnage en réponse à un gain réel et un gain caractéristique de la bobine.

5. Procédé selon la revendication 1, 2 ou 3, dans lequel le capteur de position (28) comporte au moins une bobine (60 ; 62 ; 64), et dans lequel la détermination (520) des données d'étalonnage (434), au niveau de chacune de la pluralité de fréquences, comprend l'étape consistant à déterminer les données d'étalonnage en réponse à au moins une parmi : une position de la bobine à l'intérieur du capteur de position et une orientation de la bobine à l'intérieur du capteur de position, la valeur caractéristique et la valeur réelle au niveau de chaque fréquence étant la sensibilité caractéristique (428) et la sensibilité réelle (432), respectivement.

6. Procédé selon l'une quelconque des revendications précédentes, dans lequel le capteur de position (28) comporte une pluralité de bobines (60 ; 62 ; 64), et dans lequel la détermination (520) des données d'étalonnage (434), au niveau de chacune de la pluralité de fréquences, comprend l'étape consistant à déterminer les données d'étalonnage pour chacune de la pluralité de bobines.

7. Procédé selon l'une quelconque des revendications précédentes, dans lequel le capteur de position (28) est incorporé dans un dispositif destiné à être placé chez le patient, et dans lequel la détermination (520) des données d'étalonnage (434) au niveau de chacune de la pluralité de fréquences comprend l'étape consistant à déterminer les données d'étalonnage en réponse à au moins une parmi : une position du capteur de position (28) à l'intérieur du dispositif et une orientation du capteur de position à l'intérieur du dispositif, la valeur caractéristique et la valeur réelle au niveau de chaque fréquence étant la sensibilité caractéristique (428) et la sensibilité réelle (432), respectivement.

8. Procédé selon l'une quelconque des revendications précédentes, comprenant en outre l'étape consistant à stocker (530) les données d'étalonnage (434) dans le capteur de position (28).

9. Appareil d'étalonnage d'un capteur de position (28) destiné à être placé chez un patient, l'appareil comprenant :
une installation d'essai, adaptée pour retenir le capteur de position dans une position et selon une orientation connues ;
une pluralité de bobines formant éléments rayonnants, adaptées afin de générer des champs au niveau d'une pluralité de fréquences ; et
un ordinateur (36), adapté afin de :
recevoir, pour chacune de la pluralité de fréquences, une indication d'une valeur caractéristique (428) du capteur de position,
mesurer une valeur réelle (432) du capteur de position, en réponse aux champs générés au niveau de chacune de la pluralité de fréquences, et
déterminer (520), au niveau de chacune de la pluralité de fréquences, des données d'étalonnage (434), indicatrices d'un écart de la valeur réelle par rapport à la valeur caractéristique,
**caractérisé en ce que** l'ordinateur (36) est adapté afin de déterminer les données d'étalonnage (434), au niveau de chacune de la pluralité de fréquences, en calculant par soustraction (520) une différence entre la valeur réelle (432) et la valeur caractéristique (428).

10. Appareil d'étalonnage d'un capteur de position (28) destiné à être placé chez un patient, l'appareil comprenant :
une installation d'essai, adaptée pour retenir le capteur de position dans une position et selon une orientation connues ;
une pluralité de bobines formant éléments rayonnants, adaptées afin de générer des champs au niveau d'une pluralité de fréquences ; et
un ordinateur (36), adapté afin de :
recevoir, pour chacune de la pluralité de fréquences, une indication d'une valeur caractéristique (428) du capteur de position,
mesurer une valeur réelle (432) du capteur de position, en réponse aux champs générés au niveau de chacune de la pluralité de fréquences, et
déterminer (520), au niveau de chacune de la pluralité de fréquences, des données d'étalonnage (434) indicatrices d'un écart de la valeur réelle par rapport à la valeur caractéristique,
**caractérisé en ce que** l'ordinateur (36) est adapté afin de déterminer (520), au niveau de chacune de la pluralité de fréquences, les données d'étalonnage (434) indicatrices de l'écart en exprimant l'écart en tant qu'une portion de la valeur caractéristique (428).

11. Appareil selon la revendication 9 ou 10, dans lequel l'ordinateur (36) est adapté afin de déterminer (520), au niveau de chacune de la pluralité de fréquences, les données d'étalonnage (434) indicatrices de l'écart en représentant l'écart d'une manière non linéaire en rapport avec la pluralité de fréquences.

12. Appareil selon la revendication 9, 10 ou 11, dans lequel le capteur de position (28) comporte au moins une bobine (60 ; 62 ; 64), et dans lequel l'ordinateur est adapté afin de déterminer (520) les données d'étalonnage (434), au niveau de chacune de la pluralité de fréquences, en réponse à un gain réel et un gain caractéristique de la bobine.

13. Appareil selon la revendication 9, 10 ou 11, dans lequel le capteur de position (28) comporte au moins une bobine (60 ; 62 ; 64), et dans lequel l'ordinateur est adapté afin de déterminer (520) les données d'étalonnage, au niveau de chacune de la pluralité de fréquences, en réponse à au moins une parmi : une position de la bobine à l'intérieur du capteur de position et une orientation de la bobine à l'intérieur du capteur de position, la valeur caractéristique et la valeur réelle au niveau de chaque fréquence étant la sensibilité caractéristique (428) et la sensibilité réelle(432), respectivement.

14. Appareil selon l'une quelconque des revendications 9 à 13, dans lequel le capteur de position (28) comporte une pluralité de bobines (60 ; 62 ; 64), et dans lequel l'ordinateur (36) est adapté afin de déterminer, au niveau de chacune de la pluralité de fréquences, les données d'étalonnage (434) pour chacune de la pluralité de bobines.

15. Appareil selon l'une quelconque des revendications 9 à 14, dans lequel le capteur de position (28) est incorporé dans un dispositif destiné à être placé chez le patient, et dans lequel l'ordinateur est adapté afin de déterminer (520) les données d'étalonnage (434) au niveau de chacune de la pluralité de fréquences, en réponse à au moins une parmi : une position du capteur de position à l'intérieur du dispositif et une orientation du capteur de position à l'intérieur du dispositif, la valeur caractéristique et la valeur réelle au niveau de chaque fréquence étant la sensibilité caractéristique (428) et la sensibilité réelle (432), respectivement.

16. Appareil selon l'une quelconque des revendications 9 à 15, dans lequel l'ordinateur (36) est adapté afin de stocker (530) des données d'étalonnage dans le capteur de position (28).

17. Appareil selon la revendication 16, comprenant en outre un dispositif adapté afin d'être placé chez un patient, le dispositif comprenant :
le capteur de position (28) ; et
une mémoire (91) adaptée afin de stocker des données d'étalonnage (434).

18. Appareil selon la revendication 17, dans lequel le dispositif est adapté afin d'être incorporé dans une sonde allongée (20).

19. Appareil selon la revendication 17, dans lequel le dispositif est adapté afin d'être incorporé dans une capsule (600), adaptée afin d'être placée chez le patient.
